# EUROPÄISCHE PATENTSCHRIFT

(11) **EP 1 034 292 B1**
(45) Veröffentlichungstag und Bekanntmachung des Hinweises auf die Patenterteilung: **03.07.2002**
(21) Anmeldenummer: 98965603.8
(22) Anmeldetag: 27.11.1998
(51) Int. Cl.: C12N 15/86, C12N 5/10, A61K 48/00

(54) **ZELLSPEZIFISCHE RETROVIRALE VEKTOREN MIT ANTIKÖRPERDOMÄNEN UND VERFAHREN ZU IHRER HERSTELLUNG FÜR DEN SELEKTIVEN GENTRANSFER**
CELL-SPECIFIC RETROVIRAL VECTORS WITH ANTIBODY DOMAINS AND METHOD FOR THE PRODUCTION THEREOF FOR SELECTIVE GENE TRANSFER
VECTEURS RETROVIRAUX A SPECIFICITE CELLULAIRE DOTES DE DOMAINES DE RECONNAISSANCE D'ANTICORPS ET PROCEDES DE FABRICATION DESDITS VECTEURS POUR LE TRANSFERT SELECTIF DE GENES

(30) Priorität: 28.11.1997 DE 19725854
(43) Veröffentlichungstag der Anmeldung: 13.09.2000
(73) Patentinhaber: Bundesrepublik Deutschland, letztvertreten durch den Präsidenten des Paul-Ehrlich-Instituts Prof. Dr. R. Kurth, 63225 Langen (DE)
(72) Erfinder: CICHUTEK, Klaus, D-60598 Frankfurt am Main (DE); ENGELSTÄDTER, Martin, D-63110 Rodgau (DE)
(74) Vertreter: Grund, Martin, Dr.
(86) Internationale Anmeldenummer: DE9803543
(87) Internationale Veröffentlichungsnummer: WO9928489

(56) Entgegenhaltungen:
- EP-A- 0 104 014
- WO-A-93/01288
- WO-A-95/23846
- CHU T. H. ET AL.: "Toward highly efficient cell-type- specific gene transfer with retroviral vectors displaying single-chain antibodies." JOURNAL OF VIROLOGY, Bd. 71, Nr. 1, Januar 1997, Seiten 720-725, XP002103150 in der Anmeldung erwähnt
- CHISWELL D. J. ET AL.: "PHAGE ANTIBODIES: WILL NEW 'COLICLONAL' ANTIBODIES REPLACE MONOCLONAL ANTIBODIES?" TRENDS IN BIOTECHNOLOGY, Bd. 10, Nr. 3, 1. März 1992, Seiten 80-84, XP000249633 in der Anmeldung erwähnt
- LANG I. M. ET AL.: "Recombinant rabbit Fab with binding activity to type-1 plasminogen activator inhibitor derived from a phage-display library against human alpha-granules" GENE, Bd. 172, Nr. 2, 26. Juni 1996, Seiten 295-298, XP002103151
- JIANG A. ET AL.: "Cell-type-specific gene transfer into human cells with retroviral vectors that display single-chain antibodies." JOURNAL OF VIROLOGY, Bd. 72, Nr. 12, Dezember 1998, Seiten 10148-10156, XP002103152

### SEQUENZPROTOKOLL

(1) ALLGEMEINE ANGABEN:
   (i) ANMELDER:
      (A) NAME: Bundesrepublik Deutschland letztvertreten durch den Präsidenten des Paul-Ehrlich-Instituts
      (B) STRASSE: Paul-Ehrlich-Str. 51-59
      (C) ORT: Langen
      (E) LAND: Deutschland
      (F) POSTLEITZAHL: 63225
   (ii) BEZEICHNUNG DER ERFINDUNG: Zellspezifische retrovirale Vektoren mit Antikörperdomänen und Verfahren zu ihrer Herstellung für den selektiven Gentransfer
   (iii) ANZAHL DER SEQUENZEN: 31
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (v) DATEN DER FRUEHBREN ANMELDUNG:
      ANMELDENUMMER: DE 197 52 854.6
      ANMELDETAG: 28-11-1997
(2) ANGABEN ZU SEQ ID NO: 1:
   (i) SEQUENZKENNZEICHEN:
      (A) LÄNGE: 4776 Basenpaare
      (B) ART: Nucleotid
      (C) STRANGFORM: Doppelstrang
      (D) TOPOLOGIE: linear
   (ii) ART DES MOLEKÜLS: Sonstige Nucleinsäure
   (iii) HYPOTHETISCH: NEIN
   (xi) SEGQUENZBESCHREIBUNG: SEQ ID NO: 1:
(2) ANGABEN ZU SEQ ID NO: 2:
   (i) SEQUENZKENNZEICHEN:
      (A) LÄNGE: 12 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 2:
   Met Lys Asp Pro Thr Cys Arg Phe Gly Lys Leu Ala
   5 10
(2) ANGABEN ZU SEQ ID NO: 3:
   (i) SEQUENZKENNZEICHEN:
      (A) LÄNGE: 21 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 3:
   Met Glu Lys Tyr Ile Thr Glu Asn Arg Glu Val Gln Ile Lys Val Arg
   5 10 15
   Asn Arg Trp Asn Ser
   20
(2) ANGABEN ZU SEQ ID NO: 4:
   (i) SEQUENZKENNZEICHEN:
      (A) LÄNGE: 8 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 4:
   Met Gly Gln Thr Gly Tyr Leu Trp
   5
(2) ANGABEN ZU SEQ ID NO: 5:
   (i) SEQUENZKENNZEICHEN:
      (A) LÄNGE: 13 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 5:
   Met Glu Gln Leu Asn Met Gly Gln Thr Gly Tyr Leu Trp
   5 10
(2) ANGABEN ZU SEQ ID NO: 6:
   (i) SEQUENZKENNZEICHEN:
      (A) LÄNGE: 12 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 6:
   Met Val Pro Arg Cys Gly Pro Ala Leu Ser Ser Phe
                   5                   10
(2) ANGABEN ZU SEQ ID NO: 7:
   (i) SEQUENZKENNZEICHEN:
      (A) LÄNGE: 10 Aminosäuren
      (B) ART: Aminosäure
      (D) TOPOLOGIE: linear
   (ii) ART DES MOLEKUELS: Protein
   (xi) SEQUENZBESCHREIBUNG: SEO ID NO: 7:
   Met Phe Pro Gly Cys Pro Lys Asp Leu Lys
                   5                   10
(2) ANGABEN ZU SEQ ID NO: 8:
   (i) SEQUENZKENNZEICHEN:
      (A) LÄNGE: 15 Aminosäuren
      (B) ART: Aminosäure
      (D) TOPOLOGIE: linear
   (ii) ART DES MOLEKUELS: Protein
   (xi) SEQUENZBESCHREIBUNG: SEO ID NO: 8:
   Met Val Glu Val Arg Cys Gly Arg Leu Glu Ile Trp Pro Tyr Thr
                   5                   10                  15
(2) ANGABEN ZU SEQ ID NO: 9:
   (i) SEQUENZKENNZEICHEN:
      (A) LÄNGE: 24 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 9:
   Met Thr Ser Thr Leu Pro Phe Ser Pro Gln Val Ser Thr Pro Arg Ser
                   5                   10                  15
   Asn Arg Ile Arg Ala Pro Pro Arg
               20
(2) ANGABEN ZU SEQ ID NO: 10:
   (i) SEQUENZKENNZEICHEN:
      (A) LÄNGE: 232 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 10:
   Met Asp Cys Leu Thr Asn Leu Arg Ser Ala Glu Gly Lys Val Asp Gln
                   5                   10                  15
   Ala Ser Lys Ile Leu Ile Leu Leu Val Ala Trp Trp Gly Phe Gly Thr
               20                  25                  30
   Thr Ala Glu Val Ser Thr Ala Gly Ser Gly Gly Gly Gly Ser Gly Gly
           35                  40                  45
   Gly Ser Gly Gly Gly Gly Ser Gly Gly Gly Gly Ser Gly Ala Ser Pro
       50                  55                  60
   Val Gln Phe Ile Pro Leu Leu Val Gly Leu Gly Ile Ser Gly Ala Thr
   65                  70                  75                  80
   Leu Ala Gly Gly Thr Gly Leu Gly Val Ser Val His Thr Tyr His Lys
                   85                  90                  95
   Leu Ser Asn Gln Leu Ile Glu Asp Val Gln Ala Leu Ser Gly Thr Ile
               100                 105                 110
   Asn Asp Leu Gln Asp Gln Ile Asp Ser Leu Ala Glu Val Val Leu Gln
           115                 120                 125
   Asn Arg Arg Gly Leu Asp Leu Leu Thr Ala Glu Gln Gly Gly Ile Cys
   130                 135                 140
   Leu Ala Leu Gln Glu Lys Cys Cys Phe Tyr Ala Asn Lys Ser Gly Ile
   145                 150                 155                 160
   Val Arg Asp Lys Ile Arg Lys Leu Gln Glu Asp Leu Ile Glu Arg Lys
                   165                 170                 175
   Arg Ala Leu Tyr Asp Asn Pro Leu Trp Ser Gly Leu Asn Gly Phe Leu
               180                 185                 190
   Pro Tyr Leu Leu Pro Leu Leu Gly Pro Leu Phe Gly Leu Ile Leu Phe
           195                 200                 205
   Leu Thr Leu Gly Pro Cys Ile Met Lys Thr Leu Thr Arg Ile Ile His
       210                 215                 220
   Asp Lys Ile Gln Ala Val Lys Ser
   225                 230
(2) ANGABEN ZU SEQ ID NO: 11:
   (i) SEQUENZKENNZEICHEN:
      (A) LÄNGE: 14 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 11:
   Met Asp Thr Leu Gly Val Arg Trp Ser Lys Asn Ser Arg Val
                   5                   10
(2) ANGABEN ZU SEQ ID NO: 12:
   (i) SEQUENZKENNZEICHEN:
      (A) LÄNGE: 15 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 12:
   Met Tyr Lys Ser Ser Gly Glu Cys Gly Arg Gly Leu Arg Arg Pro
                   5                   10                  15
(2) ANGABEN ZU SEQ ID NO: 13:
   (i) SEQUENZKENNZEICHEN:
      (A) LÄNGE: 16 Aminosäuren
      (B) ART: Aminosäure
      (D) TOPOLOGIE: linear
   (ii) ART DES MOLEKUELS: Protein
   (xi) SEQUENZBESCHREIBUNG: SEO ID NO: 13:
   Met Ile Arg Tyr Ile Asp Glu Phe Gly Gln Thr Thr Thr Arg Met Gln
                   5                   10                  15
(2) ANGABEN ZU SEQ ID NO:14:
   (i) SEQUENZKENNZEICHEN:
      (A) LÄNGE: 4 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 14:
   Met Leu Tyr Leu
(2) ANGABEN ZU SEQ ID NO: 15:
   (i) SEQUENZKENNZEICHEN:
      (A) LÄNGE: 6 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 15:
   Met Leu Leu Leu Tyr Leu
                   5
(2) ANGABEN ZU SEQ ID NO: 16:
   (i) SEQUENZKENNZEICHEN:
      (A) LÄNGE: 12 Aminosäuren
      (B) ART: Aminosäure
      (D) TOPOLOGIE: linear
   (ii) ART DES MOLEKUELS: Protein
   (xi) SEOUENZBESCHREIBUNG: SEO ID NO: 16:
(2) ANGABEN ZU SEQ ID NO: 17:
   (i) SEQUENZKENNZEICHEN:
      (A) LÄNGE: 26 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 17:
   Met Val Ile Ala Val Ser Cys Val Lys Leu Leu Ser Ala His Asn Ser
                    5                  10                  15
   Thr Gln His Thr Ser Arg Lys His Lys Val
               20                  25
(2) ANGABEN ZU SEQ ID NO: 18:
   (i) SEQUENZKENNZEICHEN:
      (A) LÄNGE: 49 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 18:
   Met Ser Glu Leu Thr His Ile Asn Cys Val Ala Leu Thr Ala Arg Phe
                    5                  10                  15
   Pro Val Gly Lys Pro Val Val Pro Ala Ala Leu Met Asn Arg Pro Thr
               20                  25                  30
   Arg Gly Glu Arg Arg Phe Ala Tyr Trp Ala Leu Phe Arg Phe Leu Ala
           35                  40                  45
   His
(2) ANGABEN ZU SEQ ID NO: 19:
   (i) SEQUENZKENNZEICHEN:
      (A) LÄNGE: 4 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 19:
   Met Leu Thr Leu
(2) ANGABEN ZU SEQ ID NO: 20:
   (i) SEQUENZKENNZEICHEN:
      (A) LANGE: 9 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 20:
   Met Arg Leu Ser Lys Arg Ile Phe Thr
(2) ANGABEN ZU SEQ ID NO: 21:
   (i) SEQUENZKENNZEICHEN:
      (A) LÄNGE: 11 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 21:
   Met Ser Lys Leu Gly Leu Thr Val Thr Asn Ala
                   5                   10
(2) ANGABEN ZU SEQ ID NO:22:
   (i) SEQUENZKENNZEICHEN:
      (A) LÄNGE: 70 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 22:
   Met Arg Cys Glu Ile Pro His Arg Cys Val Arg Arg Lys Tyr Arg Ile
                   5                   10                  15
   Arg Arg His Ser Pro Phe Arg Leu Arg Asn Cys Trp Glu Gly Arg Ser
               20                  25                  30
   Val Arg Ala Ser Ser Leu Leu Arg Gln Leu Ala Lys Gly Gly Cys Ala
           35                  40                  45
   Ala Arg Arg Leu Ser Trp Val Thr Pro Gly Phe Ser Gln Ser Arg Arg
       50                  55                  60
   Cys Lys Thr Thr Ala Ser
   65                  70
(2) ANGABEN ZU SEQ ID NO: 23:
   (i) SEQUENZKENNZEICHEN:
      (A) LÄNGE: 88 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 23:
   Met Ile Pro Arg Asp Pro Arg Ser Pro Ala Pro Asp Leu Ser Ala Ile
                   5                   10                  15
   Asn Gln Pro Ala Gly Arg Ala Glu Arg Arg Ser Gly Pro Ala Thr Leu
               20                  25                  30
   Ser Ala Ser Ile Gln Ser Ile Asn Cys Cys Arg Glu Ala Arg Val Ser
           35                  40                  45
   Ser Ser Pro Val Asn Ser Leu Arg Asn Val Val Ala Ile Ala Thr Gly
       50                  55                  60
   Ile Val Val Ser Arg Ser Ser Phe Gly Met Ala Ser Phe Ser Ser Gly
   65                  70                  75                  80
   Ser Gln Arg Ser Arg Arg Val Thr
                   85
(2) ANGABEN ZU SEQ ID NO: 24:
   (i) SEQUENZKENNZEICHEN:
      (A) LÄNGE: 56 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 24:
   Met Leu Cys Lys Lys Ala Val Ser Ser Phe Gly Pro Pro Ile Val Val
                   5                   10                  15
   Arg Ser Lys Leu Ala Ala Val Leu Ser Leu Met Val Met Ala Ala Leu
               20                  25                  30
   His Asn Ser Leu Thr Val Met Pro Ser Val Arg Cys Phe Ser Val Thr
           35                  40                  45
   Gly Glu Tyr Ser Thr Lys Ser Phe
       50                  55
(2) ANGABEN ZU SEQ ID NO: 25:
   (i) SEQUENZKENNZEICHEN:
      (A) LÄNGE: 49 Aminosäuren
      (B) ART: Aminosäure
      (D) TOPOLOGIE: linear
   (ii) ART DES MOLEKUELS: Protein
   (xi) SEQDENZBESCHREIBUNG: SEQ ID NO: 25:
(2) ANGABEN ZU SEQ ID NO: 26:
   (i) SEQUENZKENNZEICHEN:
      (A) LÄNGE: 6 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 26:
   Met Pro Gln Lys Arg Glu
   5
(2) ANGABEN ZU SEQ ID NO: 27:
   (i) SEQUENZKENNZEICHEN:
      (A) LÄNGE: 27 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 27:
   Met Leu Asn Thr His Thr Leu Pro Phe Ser Ile Leu Leu Lys His Leu
   5 10 15
   Ser Gly Leu Leu Ser His Glu Arg Ile His Ile
   20 25
(2) ANGABEN ZU SEQ ID NO: 28:
   (i) SEQUENZKENNZEICHEN:
      (A) LÄNGE: 5 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 28:
   Met Tyr Leu Glu Lys
   5
(2) ANGABEN ZU SEQ ID NO: 29:
   (i) SEQUENZKENNZEICHEN:
      (A) LÄNGE: 26 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 29:
   Met Thr Leu Thr Tyr Lys Asn Arg Arg Ile Thr Arg Pro Phe Arg Leu
                   5                   10                  15
   Ala Arg Phe Gly Asp Asp Gly Glu Asn Leu
               20                  25
(2) ANGABEN ZU SEQ ID NO: 30:
   (i) SEQUENZKENNZEICHEN:
      (A) LÄNGE: 11 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 30:
   Met Gln Leu Pro Glu Thr Val Thr Ala Cys Leu
                   5                   10
(2) ANGABEN ZU SEQ ID NO: 31:
   (i) SEQUENZKENNZEICHEN:
      (A) LÄNGE: 31 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 31:
   Met Pro Gly Ala Asp Lys Pro Val Arg Ala Arg Gln Arg Val Leu Ala
                   5                   10                  15
   Gly Val Gly Ala Gly Leu Thr Met Arg His Gln Ser Arg Leu Tyr
               20                  25                  30

## Beschreibung

Die Erfindung betrifft zellspezifische retrovirale Vektoren mit Antikörpererkennungsdomänen (scFv), die für die zellspezifische Transduktion eines ausgewählten Säugerzelltyps geeignet sind (Zelltargeting), Verfahren zur Herstellung der zellspezifischen retroviralen Vektoren und ihre Verwendung zur Genübertragung in ausgewählte Zellen. Die Erfindung betrifft ferner retrovirale Verpackungszellen zur Gewinnung der erfindungsgemäßen zellspezifischen retroviralen Vektoren.

Die Mehrheit der retroviralen Vektoren, die in der gentherapeutischen Forschung zur Zeit benutzt werden, stammen vom amphotropen Maus-Leukämie-Virus (MLV) ab. Der Wirtszellbereich des amphotropen MLV wird wird durch das Oberflächen-Hüllprotein (SU) bestimmt, das vom env-Gen codiert wird. Die Proteinprodukte des env-Gens bilden die äußere Hülle des retroviralen Vektors. Die SU-Proteine interagieren mit, d.h. sie binden an ein bestimmtes Protein (Rezeptor) auf der Oberfläche der Wirtszelle. Die env-Genprodukte des amphotropen MLV erlauben den Gentransfer in eine große Anzahl unterschiedlicher Säugerzellen. Ein selektiver Gentransfer in bestimmte Zell- oder Gewebetypen des Menschen oder anderer Säuger ist mit amphotropen MLV-Vektoren aber nicht möglich, weil der Rezeptor fiir die MLV-Hüllproteine auf der Oberfläche der Säugerzellen, welches den Eintritt von amphotropen MLV-Vektoren und den Gentransfer vermittelt, auf fast allen diesen Zellen zu finden ist. Der Wirtszellbereich des amphotropen MLV ist daher nicht spezifisch.

Eine Wirtszellspezifität ist z.B. für den gentherapeutischen Einsatz jedoch von Vorteil, da bei einer Gentherapie außerhalb des Organismus (*ex vivo*) (Anderson et al., *Science* 256 (1992), 808-813; Yu et al., *H. Gene Therapy* 8 (1997), 1065-1072) aufwendige Aufreinigungen von Zellen vermieden werden. Für den Therapie-, Diagnostik- oder Impf-Einsatz *in vivo* ist erwünscht, daß die retroviralen Vektoren gezielt die gewünschten Wirtszellen ansteuern und anschließend das therapeutische Gen übertragen. Eine Einengung des Wirtszellbereichs des amphotropen MLV konnte durch Modifikation des Oberflächenhüllproteins erreicht werden. Eine Modikation des Oberflächenhüllproteins wurde durch die Fusion mit einer Hormondomäne durchgeführt. Es fand eine Transduktion der Zellen statt, die den spezifischen Hormonrezeptor trugen (Kasahara et al., *Science* 266 (1994), 1373-1375). Ferner wurde das Oberflächenhüllprotein durch Fusion mit einem einkettigen Antikörperfragment (*single chain variable fragment*, nachfolgend auch "scFv" bezeichnet) modifiziert. Das Fragment repräsentierte die antigenbindende Domäne eines Antikörpers und ist ein Fusionsprotein, das aus den variablen Domänen Vh und Vl eines monoklonalen Antikörpers zusammengesetzt ist. Die beiden Domänen sind über ein Glycin- und Serin-Oligopeptid [-(ser-gly4)3-gly-)] verknüpft, das die korrekte Faltung des Fusionsproteins ermöglicht (Huston et al., *Methods Enzymol.* 203 (1991), 46-88, Whitlow et al., *Methods: A companion to Methods Enzymol.* 2 (1991), 97-105). Alle bisher durchgeführten Modifikationen des MLV-Oberflächenhüllproteins mit einem scFv zeigten, daß es zwar zu einer Bindung der Vektoren an die Wirtszielzelle kam, nicht jedoch zu einem Eintritt in die Zelle (Russel et al., *Nucleic Acid Res.* 21 (1993), 1081-1985). Weiterhin ist bekannt, daß das Oberflächenhüllprotein des MLV generell keine umfangreichen Modifikationen erlaubt (Cosset et al., *J. Virol* 69 (1995), 6314-632). Modifikationen, bei denen ein Teil der Bindungsdomäne des MLV-SU-Proteins ersetzt wurde, führten oft zu einer inkorrekten Prozessierung und somit zu einem defekten Transport des SU-Proteins an die Zelloberfläche (Weiss et al., *In J.A.Levy* (ed.). *The Retroviridae* 2 (1993), 1-108 ; Morgan et al., *J. Virol.* 67 (1993), 4712-4721; Russel et al., *Nucleic Acid Res.* 21 (1993), 1081-1985). Die Entwicklung zellspezifischer retroviraler Vektoren auf Basis des MLV mit veränderten Oberflächenhüllproteinen ist daher wenig erfolgversprechend.

Retrovirale Vektoren auf Basis des Milznekrosevirus SNV ("Spleen Necrosis Virus") sind fiir einen gezielten Gentransfer in z.B. humane Zellen geeigneter, da das Oberflächen-Hüllprotein des SNV umfangreiche Modifikationen erlaubt und auch dann noch korrekt prozessiert wird (Martinez und Dornburg, *Virol.* 208 (1995), 234-241; Chu et al., *Gene Therapie* 1 (1994), 292-299; Chu und Dornburg, *J. Virol.* 69 (1995), 2659-2663). Zur Herstellung derartiger Vektoren benötigt man mindestens zwei Komponenten. Zum einen ist ein sog. Expressionskonstrukt herzustellen, das eine Verpackung in und den Transfer durch einen Retrovirus erlaubt. Das Expressionskonstrukt umfaßt ein kodierendes DNA-Fragment des gewünschten Genprodukts, z.B. ein Gen für die Gentherapie oder als Impfstoff. Das Expressionskonstrukt muß eine Nukleotidsequenz umfassen, die als Verpackunssignal psi (ψ) bezeichnet wird und die effiziente Verpackung der mRNA in retrovirale Partikel steuert. Ferner benötigt man eine Verpackungs- oder Helferzelle, welche die gag-, pol- und env-Genprodukte des SNV bereitstellt, ohne daß die gag-, pol- und env-Gene in ein Retrovirus verpackt werden können. Die in der Verpackungszelle befindlichen gag-, pol- und env-Gene müssen psi-negativ sein. Nach Überführung des Expressionskonstruktes durch Transfektion der entsprechenden Plasmid-DNA in die Verpackungszellen werden retrovirale Partikel in den Zellkulturüberstand abgegeben, die das Expressionskonstrukt enthalten und nur dieses, nicht jedoch die gag-, pol-, und env-Gene in die Zielzelle überführen können. Diese Vektoren sind vermehrunsunfähig und durchlaufen lediglich eine Replikationsrunde. Das allgemeine Verfahren zur Herstellung von vermehrungsunfähigen retroviralen Vektoren ist Stand der Technik (Weiss et al., *In J.A.Levy* (ed.). *The Retroviridae* 2 (1993), 1-108 ; Morgan et al., *J. Virol.* 67 (1993), 4712-4721; Russel et al., *Nucleic Acid Res.* 21 (1993), 1081-1985; Cosset et al., *J. Virol* 69 (1995; Martinez und Dornburg, *Virol.* 208 (1995), 234-241; Chu et al., *Gene Therapie* 1 (1994), 292-299; Chu und Dornburg, *J. Virol.* 69 (1995), 2659-2663).

Auch der Tropismus (Wirtszellspezifität) des Milznekrosevirus wird durch das Oberflächenhüllprotein (SU-Protein) bestimmt, das vom env-Gen des SNV codiert wird. Das Wildtyp-SNV-Oberflächenhüllprotein läßt keinen selektiven Gentransfer in bestimmte Zellen oder Gewebe des Menschen zu, da das spezifische Empfängerprotein (Rezeptor) nicht auf der Oberfläche von humanen Zellen vorhanden ist (Dornburg, *Gene Therapie* 2 (1995), 1-10). Deshalb wurde ein Verfahren entwickelt, um das SU-Protein des SNV gegen die antigenerkennende Domänen von Antikörpern zu ersetzten. Diese [SNV-scFV-Env]-Vektoren mit den zwei bisher bekannten unterschiedlichen scFv waren in der Lage, das psi-positive Reportergen, die bakterielle β-Galaktosidase, in die ausgewählte humanen Zielzellen zu übertragen. Diese scFv sind gegen das Hapten Dinitrophenol (DNP) bzw. gegen ein unbekanntes Oberflächenmolekül auf Colon-CA-Zellen und anderen Krebszellen gerichtet. (Chu et al., *Gene Therapie* 1 (1994), 292-299; Chu et al., *BioTechniques* 18 (1995), 890-899; Chu und Dornburg, *J*. *Virol.* 71 (1997), 720-725). Es wurde eine Verpackungszellinie (DSH-CXL) entwickelt, die sowohl die psi-negativen SNV-Gene gag, pol und env als auch das psi-positive Reportergen-Expressionskonstrukt (pCXL) enthält. Nach Transfektion der Verpackungszelle mit der Plasmid-DNA eines weiteren env-Expressionsgens (pTC53), bei dem das gesamte Oberflächen-Hüllprotein gegen ein einkettiges Antikörperfragment (scFv) ersetzt wurde, wurden retrovirale Vektoren in den Zellüberstand abgegeben, die auf ihrer Oberfläche neben dem Wildtyp-Oberflächenhüllprotein auch das chimäre [scFv-Env]-Oberflächenprotein trugen. Mit Hilfe dieser Vektoren konnte das Reportergen in die fiir die scFv-spezifischen Zielzellen, Hundeosteosarkomzellen (D17) die mit DNP konjugiert waren, bzw. HeLa-Zellen (humane Cervixkarzinomzellen) transferriert werden. Bei diesem beschriebenen Verfahren zur Herstellung zellspezifischer retroviraler Vektoren ist jedoch von Nachteil, daß nur bereits bekannte und klonierte scFv verwendet werden können. Ferner wurde von uns festgestellt, daß nicht jedes scFv als Teil eines [SNV-scFv-Env]-Vektors für die Zelltransduktion (Übertragen des gewünschten Gens auf die Zielzelle) geeignet ist.

Der Gentransfer in Säugerzellen mittels Retroviren hat generell folgende Vorteile:
- Es wird in der Regel eine Kopie des gewünschten Gens in die Säugerzelle überführt.
- Das gewünschte Gen wird im allgemeinen ohne Mutation oder Rearrangements übertragen.
- Es erfolgt ein stabiler Einbau des gewünschten Gens in das Genom der Zielzelle.
Weiterhin ist erwünscht, daß der retrovirale Vektor eine bestimmte Zellspezifität besitzt, durch die das z.B. therapeutische Gen in eine ausgewählte Zellpolpulation eingeführt werden kann.

Der Erfindung liegt daher die Aufgabe zugrunde, zellspezifische retrovirale Vektoren mit Antikörpererkennungsdomänen für den selektiven Gentransfer in Säugerzellen sowie ein universelles Verfahren zu ihren Herstellung bereitzustellen. Mit Hilfe dieser Vektoren gelingt es, den Gentransfer zu verbessern. Der Erfmdung liegt ferner die Aufgabe zugrunde retrovirale Verpackungszellen zur Gewinnung der erfindungsgemäßen Vektoren bereitzustellen. Die Lösung dieser Aufgaben ergibt sich aus den Patentansprüchen, der nachfolgenden Beschreibung und den Figuren.

Die Aufgabe wird durch das erfindungsgemäße Verfahren zur Herstellung zellspezifischer retroviraler Vektoren gelöst, umfassend die folgenden Schritte: a) Immunisieren eines Säugetiers mit einer oder mehreren Zellpopulation(en), b) Isolieren von RNA aus dem immunisierten Säugetier, umfassend die B-Zell-RNA, c) Herstellen von cDNA-Abschnitte der variablen Regionen der schweren und leichten Kette der Immunglobuline aus der isolierten RNA mittels RT-PCR mit Primern für die schwere und leichte Kette der Immunglobuline, wobei die Primer die Nukleinsäuresequenz für einen Oligopeptidlinker umfassen, d) Ligieren der cDNA-Abschnitte zu scFv-cDNAs, e) Ligieren der scFv-cDNAs in einen Phagemid-Vektor und Transformieren eines Wirtsbakteriums mit dem Phagemid-Vektor, f) Isolieren von Phagen, die an die in Schritt a) verwendete(n) Zellpopulation(en) binden, durch Selektion, g) Isolieren von zellspezifischen Phagen aus den in Schritt f) erhaltenen Phagen, die nur an die in Schritt a) verwendete(n) Zellpopulation(en) binden, durch Selektion, h) Ausschneiden der scFv-codierenden DNA-Fragmente aus den in Schritt g) erhaltenen zellspezifischen Phagen und Ligieren in einen psi-negativen retroviralen Env-Expressionsvektor, i) Transformieren des erhaltenen Env-scFv-Expressionsvektors in eine Verpackungszelle, und j) Isolieren der von der Verpackungszelle sezernierten retroviralen Vektoren.

Gegebenenfalls umfaßt das erfindungsgemäße Verfahren weiter die Vereinzelung der in Schritt g) erhaltenen zellspezifischen Phagen. Gegebenenfalls umfaßt das erfindungsgemäße Verfahren weiter den Schritt: k) Isolieren der von der Verpackungszelle sezernierten retroviralen Vektoren, die die Zellen der Zellpopulation(en) transduzieren durch Selektion. Ferner können die Schritte f) und/oder g) mindestens einmal wiederholt werden.

Bevorzugt ist ein Verfahren, wobei das immunisierte Säugetier ausgewählt ist aus der Gruppe Maus, Ratte, Meerschweinchen, Kaninchen, Ziege oder Schaf. Bevorzugt ist ebenfalls ein Verfahren, bei dem die Zellpopulation(en) ausgewählt ist aus der Gruppe Mensch, Maus, Ratte, Schaf, Rind oder Schwein. Besonders bevorzugt ist ein Verfahren, bei dem die Zellpopulation(en) ausgewählt ist (sind) aus der Gruppe T-Zellen, Epithelzellen, Muskelzellen, Stammzellen, neurale Zellen, hämatopoietische Zellen, Karzinomzellen oder Leberzellen. Bevorzugt ist ein Verfahren, bei dem das env-Gen vom Milznekrosevirus (SNV) stammt. Besonders bevorzugt ist ein Verfahren, bei dem der Expressionsvektor der Vektor mit der Bezeichnung pTC53 ist.

Die mit dem erfindungsgemäßen Verfahren erhältlichen zellspezifischen retroviralen Vektoren können als Arzneimittel verwendet werden. Bevorzugt ist die Verwendung zur Herstellung eines Arzneimittels zur Gentherapie, Impftherapie oder Diagnostik. Besonders bevorzugt ist die Therapie der Cystischen Fibrose, des ADA-Mangels, von HIV-Infektionen, Leukämie, chronischer Granulomatose.

Die Erfindung wird ferner glöst durch die Bereitstellung von retroviralen Verpackungszellen zur Gewinnung der erfindungsgemäßen retroviralen Vektoren, transformiert sowohl mit einem oder mehreren psi-negativen Expressionskonstrukt(en), die die gag-, pol- und/oder env-Genprodukte exprimieren, als auch mit einem psi-negativen Env-scFv-Expressionskonstrukt nach Anspruch 1 h). Bevorzugt ist eine Verpackungszelle, ferner umfassend ein psi-positives Expressionskonstrukt, umfassend ein Nukleinsäurefragment, das in die durch den retroviralen Vektor zu transduzierende Zelle eingeführt werden soll. Besonders bevorzugt ist eine Verpackungszelle, wobei das Nukleinsäurefragment ein therapeutisches Gen oder dessen DNA-Fragment und/oder ein Reportergen umfaßt. Insbesondere bevorzugt ist eine Verpackungszelle, wobei das therapeutische Gen oder dessen Nukleinsäurefragment das CFTR-, phox91-, ADA-, IL-16-, p53- oder revM10-Gen oder Impfgene z. B. rekombinantes gp120 und IL-16 umfaßt. Ferner insbesondere bevorzugt ist eine Verpackungszelle, wobei das Reportergen β-Galaktosidase, "Green Fluorescent Protein", Luciferase oder Neomycin umfaßt.

### Die Abbildungen dienen der Erläuterung der Erfindung

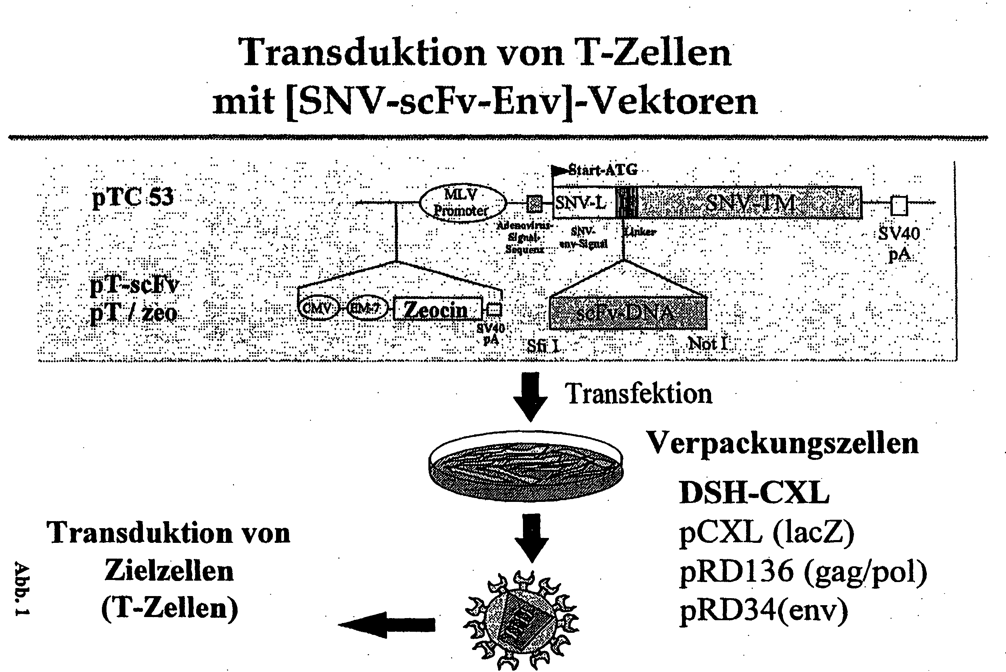

Abb.1 zeigt schematisch die Env-scFv Expressionskonstrukt pTC53, pT-scFv und pT/zeo, deren Transfektion in die Verpackungszelle DSH-CXL, die die erfindungsgemäßen Vektoren sezerniert.

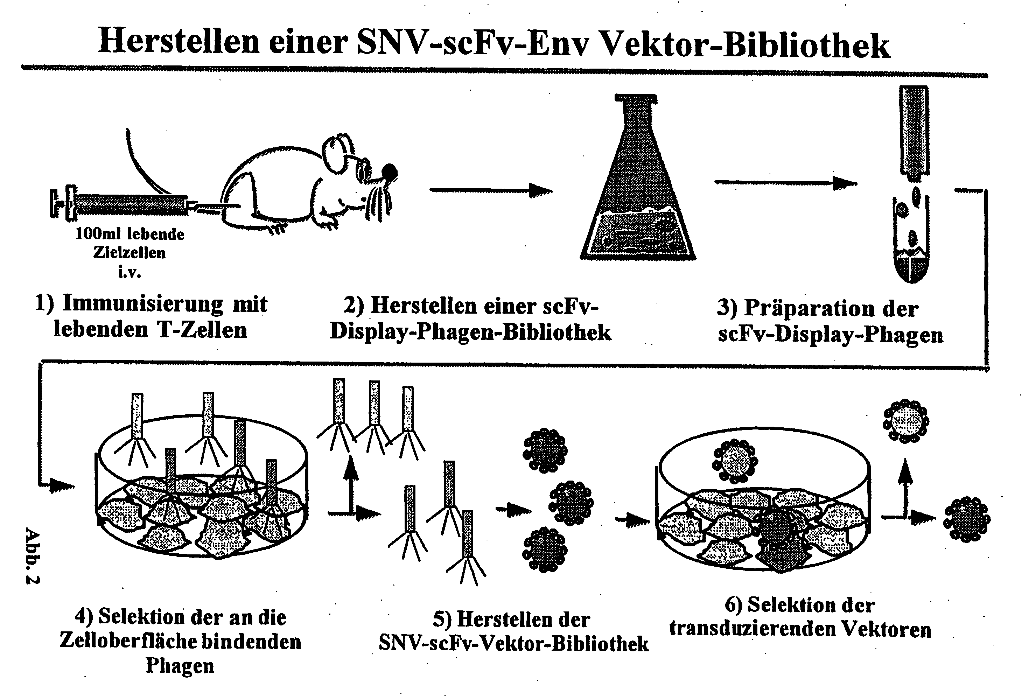

Abb.2 zeigt schematisch die Herstellung, Isolierung und Selektion der erfindungsgemäßen Vektoren.

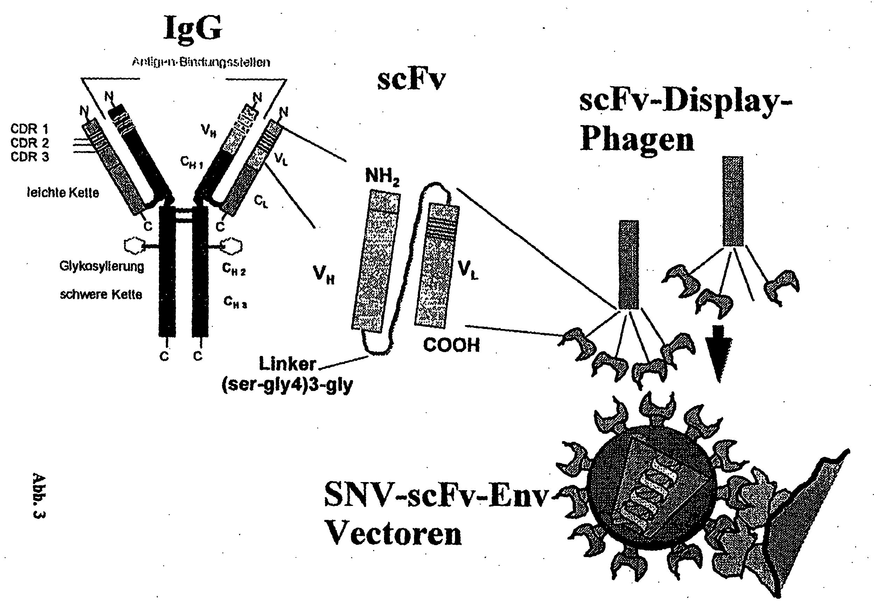

Abb.3 ist eine schematische Darstellung eines Immunglobulins und das daraus resultierende scFv. Dargestellt sind weiterhin schematisch scFv-Display-Phagen und SNV-scFv-Env-Vektoren
Abb. 4 zeigt die Nukleinsäuresequenz von pTC53.

Der hier verwendete Begriff amphotropes Virus bedeutet Infektion und Replikation auf murinen und humanen Zellen, im Gegensatz zu ecotropen Viren, das nur auf murinen Zellen repliziert. Der hier verwendete Begriff retroviraler Vektor bedeutet replikationsdefizientes retrovirales Viruspartikel, das anstelle der retroviralen mRNA eine fremde eingeführte RNA eines Gens, z.B. eines therapeutischen Gens oder dessen Fragment oder eines Reportergens übertragen kann. Der hier verwendete Begriff Antikörpererkennungsdomäne (scFv) bedeutet Antigenbindestelle eines Antikörpers, umfassend Vh- und Vl-Kette. Der hier verwendete Begriff SNV bedeutet Milznekrosevirus mit seinen Stämmen und Substämmen. SNV gehört zu den Reticolo Endotheliose Viren (REV) der Vögel, Typ D-Retrovirus.

Für die Bereitstellung der zellspezifischen Antikörpererkennungsdomänen (scFv) wird eine neue kombinatorische Phagen-cDNA-Bibliothek der variablen Domänen der leichten und schweren Ketten der Immunglobuline hergestellt. Dazu wird ein Säugetier, z.B. eine Maus, Ratte, ein Kaninchen, Meerschweinchen, Ziege oder Schaf, mit einem ausreichenden Titer einer oder mehrerer Zellpopulation(en) in üblicher Weise immunisiert. Die Zellpopulation ist die Zellart, die einen Oberflächenrezeptor ausbildet, an den die erfindungsgemäßen retroviralen Vektoren spezifisch binden. Die Zellen können von einem von dem zu immunisierenden Säugetier verschiedenen Säugetier stammen, z.B. vom Menschen, der Maus, Ratte, dem Schaf, Rind oder Schwein. Die Zellen können solche Zellen sein, in der z.B. eine somatische Gentherapie, eine Impftherapie oder Diagnostik durchgeführt werden soll. Typische Beispiele solcher Zellen sind T-Zellen, Leberzellen, Muskelzellen, neurale Zellen, Fibroblasten, Epithelzellen, Stammzellen oder hämatopoietische Zellen. Für die Immunisierung kann eine Zellpopulation oder mehrere Zellpopulationen gleichzeitig dem Säugetier verabreicht werden, je nachdem für welche Zellpopulationen(en) der erfindungsgemäße retrovirale Vektor spezifisch sein soll.

Für die Herstellung der cDNA-Bibliothek wird zuerst die B-Zell-RNA des immunisierten Säugetiers in bekannter Weise isoliert. Die mRNA-Sequenzen der für die Antigenerkennung verantwortlichen Regionen der schweren und leichten Kette (V_{H} und V_{L}) der Immunglobuline werden mittels reverser Transkription und anschließender Polymerase-Kettenamplifikation in üblicher Weise in cDNA umgeschrieben und vervielfältigt. Die Primerpaare und deren Sequenzen für die V_{H}- und V_{L}-Regionen sind dem Fachmann bekannt Sie sind z.B. im kommerziell erhältlichen Kit der Fa. Pharmacia enthalten, bzw. können den bekannten Datenbanken (EMBL) entnommen werden. Dem Fachmann ist bekannt, daß er fiir jede immunisierte Säugetierart verschiedene Primersequenzen verwenden muß. Die Sequenzen sind ebenfalls in den bekannten Datenbanken enthalten.. Die cDNA-Fragmente der V_{H}- und V_{L}-Regionen werden dann mittels einer Ligasereaktion in üblicher Weise zu scFv-cDNAs verknüpft. Für den Fachmann ist ersichtlich, daß bei der Ligation unterschiedliche Kombinationen von cDNA-Fragmenten hergestellt werden. Die erhaltenen scFv-cDNAs können dann in einen Phagemid-Vektor z.B. pCANTA 5E Phagemid, Fa. Pharmacia kloniert werden. Anschließend werden Wirtsbakterien z.B. E.coli TG1 mit dem Phagemid-Vektor transformiert.

Die von den Bakterien produzierten rekombinanten Phagen werden dann in üblicher Weise isoliert und auf das Vorhandensein von zellspezifischen scFv-Peptiden selektioniert. Die Phagen werden mit der Zellpopulation oder den Zellpopulationen in üblicher Weise in Kontakt gebracht, die für die Immunisierung verwendet worden sind. Die Phagen, die nicht an die Zellen binden, tragen kein spezifisches scFv-Peptid und werden durch Waschschritte in üblicher Weise entfernt. Die Phagen, die an die Zellen binden, präsentieren das gewünschte scFv-Peptid auf ihrer Oberfläche und werden in üblicher Weise eluiert. Die Pagen, die das gewünschte scFv-Peptid präsentieren, werden vermehrt, indem man sie wieder in üblicher Weise die Wirtsbakterien infizieren läßt. Dieser Selektionsschritt kann ein oder mehrere Male wiederholt werden, um die bindenden Phagen anzureichern. Dieser Vorgang wird als "panning" bezeichnet. Die Phagen werden nach dem panning oder direkt nach dem ersten Selektionsschritt einer weiteren Selektion unterzogen. Dabei werden die Phagen mit einer oder mehreren anderen Zellpopulationen in Kontakt gebracht, die sich von den zur Immunisierung verwendeten Zellen unterscheiden. Die Phagen, die nicht an diese Zellen binden, präsentieren ein zellspezifisches scFv-Peptid. Sie werden in üblicher Weise aus dem Zellüberstand isoliert und fiir eine Wirtsbakterieninfektion zur Vermehrung verwendet. Auch dieser Selektionsschritt kann ein oder mehrere Male wiederholt werden (Marks et al., *Biotechnologie* 10 (1992), 779; Clackson et al., *Nature* 352 (1991), 624; Marks et al., *J. Mol. Biol.* 222 (1991), 581; Chaudhary et al., *Proc. Natl. Acad. Sci. USA* 87 (1990), 1066; Chiswell et al., *TIBTECH* 10 (1992), 80; McCafferty et al., *Nature* 348 (1990), 552; Huston et al., *Proc. Natl. Acad. Sci. USA* 85 (1988), 5879).

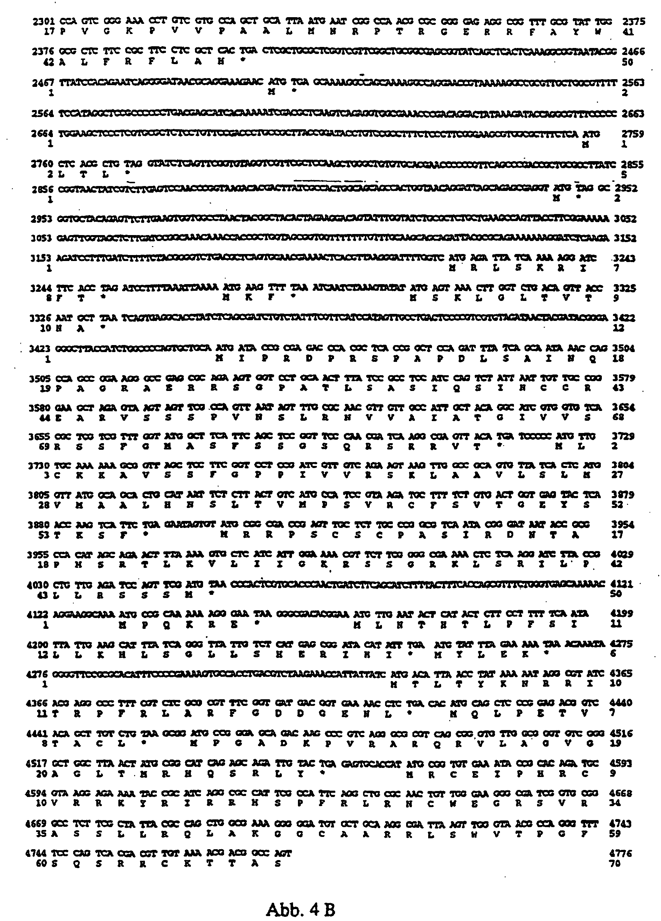

Zur Herstellung der erfindungsgemäßen zellspezifischen retroviralen Vektoren dient die vorstehend beschriebene Phagen-cDNA-Bibliothek als Ausgangsmaterial. Die scFv-cDNAs der Phagen, die nach dem zweiten Selektionsschritt und den gegebenenfalls durchgeführten panning-Verfahren übriggeblieben sind, werden in üblicher Weise aus der Phagen-DNA ausgeschnitten und in ein retrovirales Env-Expressionsgen inseriert. Das retrovirale env-Expressionsgen kann vom SNV stammen. Ein typisches Beispiel für ein SNV-scFv-env-Expressionskonstrukt ist pTC53. Die Sequenz ist in Abbildung 4B gezeigt. Ein typisches Beispiel fiir ein Wildtyp(wt)-SNV-env-Expressionskonstrukt ist pIM29.

Die Konstruktion der für die wt-SNV-ENV-Proteine z.B. pIM29 und die chimären SNV-scFv-ENV-Proteine kodierenden Expressionplasmide ist von Chu et al. (*J. Virol.* 71 (1997), 720-725) vorbeschrieben. Die Expression der für das wt-env-Gen kodierenden DNA wird von einem MLV-Promotor gesteuert. Die env-cDNA wurde über die Restriktionsschnittstellen SacII und AvrII aus einem fiir das komplette SNV-Virus kodierenden Plasmids ausgeschnitten und durch Insertion in einen Linker (L) eingefügt. Um eine korrekte Prozessierung des Proteins zu gewährleisten, enthält pIM29 die Polyadenylierungsstelle des Simianen Virus 40 (SV40). Von diesem Plasmid kann somit die Expression des wt-env-Gens erfolgen, so daß nach proteolytischer Spaltung eines Vorläuferproteins das äußere Glykoprotein (SU) und das Transmembranprotein (TM) vorliegt. Es können jedoch andere, dem Fachmann bekannte Plasmide, Promotoren, Linker, Polyadenylierungssignale und weitere für eine korrekte Prozessierung benötigte DNA-Elemente verwendet werden.

Zur Exprimierung von SNV-scFv-ENV-Proteinen werden die in bekannter Weise erhaltenen scFv in ein SNV-ENV-Expressionskonstrukt z.B. pTC53 in üblicher Weise eingeführt. Die in pTC53 vorhandenen Restriktionserkennungstellen für die Enzyme SfiI und NotI ermöglichen die molekulare Klonierung von z.B. scFv zwischen SNV-env-Leader-Sequenz und dem für das Transmembranprotein (TM-Protein) kodierenden Bereich der DNA. Die im wt-ENV vorhandene Proteaseschnittstelle zwischen SU und TM ist in pTC53 deletiert, so daß ein Fusionsprotein exprimiert wird, das N-terminal aus dem einkettigen Antikörperframgent und C-terminal aus dem SNV-TM besteht. Die regulatorischen Elemente wie MLV-Promotor und SV40-Polyadenylierungssignal sind identisch mit denen des pIM29-Vektors. Zur Verstärkung der Expression eines chimären env-Gens wird in dem Expressionsplasmid pTC53 eine adenovirale Leader-Sequenz z.B. AVtl (Sheay et al. *BioTechniques*, 15 (1993), 856-861) inseriert. Eine Zoezin-Kasette (pSV2zeo; Fa. Invitrogen, Niederlande) dient der möglichen Selektionierung von stabil transfizierten Zellen, so daß einzelne Zellklone etabliert werden können.

Das psi-negative SNV-scFv-env -Expressionskonstrukt kann mittels Elektroporation oder anderer bekannter Verfahren in üblicher Weise in Verpackungszellen eingeführt werden. Eine typische Verpackungszelle ist z.B. DSH-CXL. Die Verpackungszellen haben ferner psi-negative env, gag, pol-Expressionskonstrukte und für den gewünschten Gentransfer in die spezifischen Zielzellen ein weiteres psi-positives Expressionskonstrukt, das z.B. ein Gen oder DNA-Fragment fiir die Gentherapie, Impftherapie oder ein Reportergen für die Diagnostik umfaßt. Nach Transfektion der Verpackungszellen kommt es zu einer transienten Expression und Abgabe der retroviralen Vektoren, die neben natürlichen SU-Proteinen rekombinante SU-scFv-Proteine präsentieren, in den Zellkulturüberstand. Die retroviralen Vektoren können dann in üblicher Weise zur Transduktion der Zielzelle, d.h. der Zellpopulation, die zur Immunisierung verwendet wurde, eingesetzt werden. Gegebenenfalls kann dieser Schritt ein weiterer Selektionsschritt sein. Nur die erfindungsgemäßen retroviralen Vektoren, die die Zielzelle in ausreichender Weise transduzieren, werden weiter verwendet. Diese Vektoren können einem weiteren Selektionsschritt unterzogen werden. Die Vektoren können in üblicher Weise zur Transduktion von anderen Zellpopulationen als der Zellpopulation, mit der das Säugetier immunisiert wurde, eingesetzt werden. Die Vekoren, die nicht die anderen Zellen transduzieren, sondern nur die Zielzellen, können also in einem doppelten Selektionsschritt erhalten werden.

Für die Etablierung von stabilen Verpackungszellinien, welche die erfindungsgemäßen retroviralen Vektoren konstitutiv abgeben, kann ein Selektionsmarker, z.B. das Zeocin-Resistenzgen (Fa. Invitrogen), in üblicher Weise in das scFv-Expressionskonstrukt z.B. pTC53 inseriert werden. Die mit dem Zeocin-Resistenz-Gen versehenen scFv-Expressionskonstrukte werden mittels z.B. der Liposomen-Technik (Lipofectamin, Gibco BRL) in die Verpackungszellen transferriert. Nach einer ca. zwei-wöchigen Selektion der Transfektanden in Zeocin-haltigem Kulturmedium können Zellklone etabliert werden, die je nach scFv-cDNA-Fragment Ziekellpopulationen mit einem Titer von etwa 10⁴-10⁶ retroviralen Vektoren pro ml transduzierten.

Das mit den erfindungsgemäßen retroviralen Vektoren in die Zielzellpopulation oder - populationen transduzierte Gen kann z.B. die RNA eines therapeutischen Gens oder dessen Fragment sein. Therapeutische Gene können z.B. das CFTR-Gen, das ADA-Gen, der LDL-Rezeptor, β-Globin, Faktor VIII oder Faktor IX, das Dystrophin-Gen sein. Im Falle des CFTR-Gens wären die Zielzellen z.B. die Lungenepithelzellen, beim ADA-Gen die Stammzellen des Knochenmarks oder T-Lymphocyten, beim LDL-Rezeptor die Leberzellen, beim Dystrophin-Gen die Skelettmuskelzellen, beim β-Globin-Gen die hämopoietischen Stammzellen, beim Faktor VIII oder Faktor IX die Fibroblasten und Leberzellen. Dem Fachmann ist ersichtlich, daß diese Aufzählung nur eine Auswahl der therapeutischen Gene darstellt und andere Gen ebenfalls fiir eine Gentherapie verwendet werden können. Die DNA-Fragmente eines therapeutischen Gens umfassen z.B. Antisense-Nukleinsäuren oder Ribozyme. DNA-Fragmente können ferner Bereiche eines Gens umfassen, die die Trinukleotidwiederholungen von z.B. des Fragile X Gens enthalten.

Ferner kann die RNA eines Reportergens, z.B. β-Galaktosidase, GFP, Luciferase oder Neomycin in die erfindungsgemäßen retroviralen Vektoren eingeführt werden. Die Reportergene ermöglichen, festzustellen, ob die Zielzellen mit den retroviralen Vektoren transduziert worden sind.

Ferner kann die RNA eines Gens oder dessen Fragment zu Impfzwecken in die Zielzelle transduziert werden. Ein typisches "Impfgen" ist z.B. das rekombinante gp120 oder gp160 von HIV. Die Transduzierung von Immunzellen mit diesen Genen oder Fragmenten regt zu einer Antikörperbildung gegen die viralen Genprodukte an.

Die erfindungsgemäßen Vektoren können z.B. durch i.v-. oder i.m-Injektionen appliziert werden. Es können jedoch auch die erfindungsgemäßen Verpackungszellen in z.B. Organoide (Teflonbeutel) eingeschlossen werden, die dann in den Organismus eingepflanzt werden und in die Blutbahn oder ins Gewebe die erfindungsgemäßen Vektoren sezernieren. Weitere Applikationsformen sind für den Fachmann offensichtlich.

Die erfindungsgemäße retrovirale Verpackungszelle zur Gewinnung der erfindungsgemäßen pseudotypisierten retroviralen Vektoren wird bereitgestellt, indem eine Zellinie, z.B. eine humane Zellinie mit den psi-negativen Expressionskonstrukten, die die gag- und pol-Genprodukte des SNV exprimieren, und mit dem psi-negativen SNV-Env-Expressionskonstrukt und/oder psi-negativen SNV-scFv-env-Expressionskonstrukt, in üblicher Weise transfiziert wird.

Ferner können Verpackungszellen verwendet werden, die bereits die psi-negativen Expressionskonstrukte für die gag und pol-Genprodukte enthalten. In solche Verpackungszellen müssen dann nur das psi-negative Expressionskonstrukt für die Virushülle und das psi-positive Expressionskonstrukt für die in die Zielzelle zu transduzierende Nukleinsäuresequenz transfiziert werden. Dem Fachmann sind die Verfahren zur Transfektion der Expressionskonstrukte bekannt. Von den erfindungsgemäßen Verpackungszellen werden retrovirale Vektorpartikel in den Zellüberstand abgegeben, die das Expressionskonstrukt enthalten nicht jedoch die Konstrukte, die für die GAG-, POL- und ENV-Proteine kodieren. In die Zielzelle wird somit nur das gewünschte z.B. therapeutische Gen oder Reportergen überführt.

Die dargestellte Erfindung eröffnet die folgenden Möglichkeiten:
- Gene, Gen-Fragmente oder sonstige Nukleinsäuresequenzen können in ausgewählte Säugerzellen überführt werden,
- weitere Effizienzsteigerungen des Nukleinsäuretransfers durch Verbesserung der env-Genkonstrukte können erreicht werden,
- Gentherapie-, Markierungs- und Impfstrategien können entwickelt werden, fiir die ein selektiver Nukleinsäuretransfer in ausgewählte Säugerzellen wünschenswert ist.

Die folgenden Beispiele erläutern die Erfindung und sind nicht als einschränkend aufzufassen:

### 1. Isolierung und Klonierung zellspezifischer scFv

Zur Herstellung, Isolierung und Selektion von zellspezifischen scFv wurde eine Maus mit der humanen T-Zellinie T-C8166 (Clapham et al., *Virology* 158 (1987), 44-51) in üblicher Weise immunisiert, die Milz entfernt und die RNA isoliert. Die Klonierung der scFv-cDNAs wurde mit dem kommerziell erhältlichen Kit der Fa. Pharmacia nach Angaben des Herstellers durchgeführt. Die erhaltenen Phagen wurden in üblicher Weise auf ihre Bindungseigenschaften gegenüber den Zielzellen untersucht. Es wurden 150 Phagen isoliert, die spezifisch an die Zielzellen banden. Die 150 so erhaltenen zellspezifischen scFv wurden verwendet, um die erfindungsgemäßen SNV-scFv-Vektoren herzustellen.

### 2. Klonieren der spezifischen scFv-cDNA-Fragmente in Env-Expressionskonstrukte

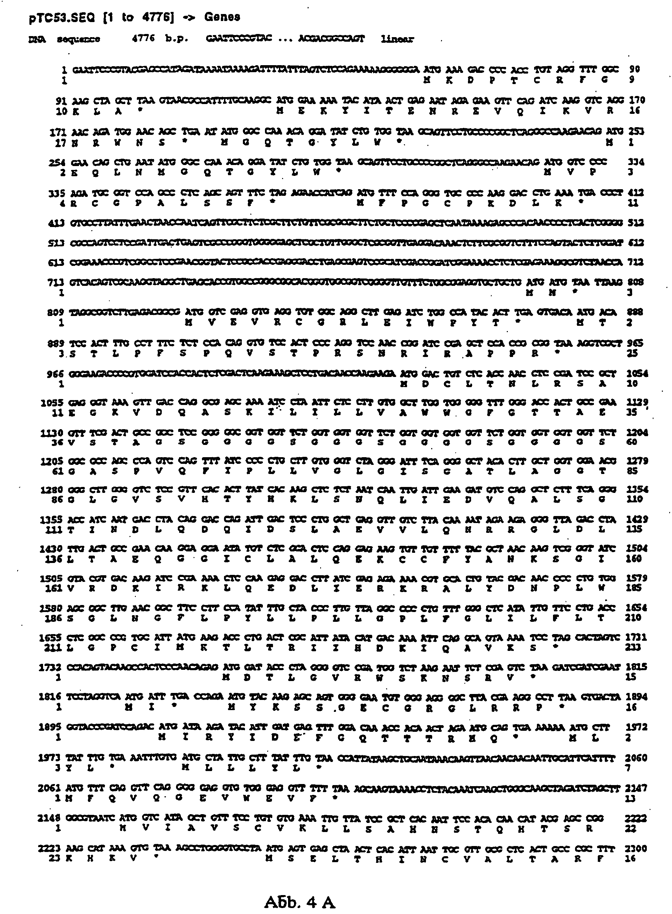

Die scFv-cDNAs der 150 zelispezißschen scFv wurden in üblicher Weise aus der Phagemid-DNA ausgeschnitten und jeweils in das Expressionskonstrukt pTC53 ligiert. pTC53 wurde erhalten durch Modifzierung des universellen eukaryotischen Vektors pRD114 (Chu et al., *J. Virol.* 71 (1997), 720-725; Sheay et al. *BioTechniques*, 15 (1993), 856-861; Chu et al., *BioTechniques*, 18 (1995). 890-895). In diesem Vektor wurde das SNV-wt-env-Gen bis auf die für die Leader-Sequenz und das Transmembran-Protein kodierende c-DNA deletiert. Ein zusätzlich eingeführter Spacer ermöglicht die Insertion einer Fremd-DNA (hier die scFv-cDNA) im Anschluß an die ENV-Leader-Sequenz über die Restriktionserkennungsstelle NaeI. Die Sequenz von pTC53 ist in Abbildung 4 gezeigt. Für die Insertion der scFv-cDNA wurde das Env-Expressionskonstrukt pTC53 dahingehend modifiziert, daß Sfi I und Not I spezifische Restriktionsendonuklease-Erkennungsstellen in den Linker zwischen der SNV-Leader-Sequenz und der SNV-Transmembran-Sequenz (TM) in üblicher Weise eingefügt werden. Hierzu wird eine rekombinante PCR ausgehend von der DNA des Plasmids PKA1558 (Scov H. & Andersen K.B., 1993) und der fiir das anti Transferrinrezeptor-scFv kodierenden DNA in üblicher Weise durchgeführt, so daß über Nru I (5'und 3') eine Insertion des amplifizierten Fragments in das Nae I restringierte pTC53 möglich ist. Das so inserierte Fragment enthält die multiple Sfi I / Not I Klonierungsstelle, da die verwendeten Primer neben der endständigen Nru I Erkennungsstelle weiterhin eine benachbarte Sfi I bzw. Not I Erkennungsstelle beinhalten. Für die rekombinante PCR wurden folgende Primer verwendet

Die PCR-Bedingungen waren: 94°C/3 min, 94°C/1 min, 59°C/1 min, 72°C/1 min., 25 X Schleifen, 72°C/10 min und dann bis 4°C abkühlen. Das PCR-Fragment wurde gelelektrophoretisch aufgetrennt, aus der Gelmatrix extrahiert (Quiaex, Fa. Quiagen) und mit dem Nae I geöffneten Plasmid pTC53 in üblicher Weise ligiert.

Die scFv-cDNAs aus dem Phagemid (pCANTA 5E) wurden mittels der Restriktionsendonukleasen Sfi I and Not I ausgeschnitten. Dazu wurde Phagemid-Plasmid-DNA mittels bekannter Verfahren hergestellt. und jeweils 8µg Plasmid-DNA mit jeweils 60 U der Restriktionsendonukleasen Sfi I und Not 1 für 1,5h bei 50°C und anschließend 1,5h bei 37°C verdaut. Der Reaktionsansatz fand in einem Volumen von 200µl statt, der mit 20µl BSA (10fach konz.) und 20µl Reaktionspuffer 3 (10fach konz.) supplementiert wurde. Nach Beendigung der Reaktionszeit wurde der Ansatz in einem 1%igem Agarosegel elektrophoretisch aufgetrennt. Nach der Auftrennung wurde die scFv-cDNA spezifische Bande (ca 750bp) aus dem Agarosegel mittels bekannter Verfahren aufgereinigt.

Das aufgereinigte Fragment wurde mit dem ebenfalls mit den Reastriktionsendonukleasen Sfi I und Not I geöffneten Env-Expressionskonstrukt pTC53 ligiert. Dazu wurden äquimolare Mengen des scFv-cDNA-Fragments und pTC53-Fragments in einem 15µl Volumen mit 200 U T4-Ligase und 1,5µl 10-fach Ligase-Puffer supplementiert. Der Ansatz wurde bei 4°C Über Nacht inkubiert. Um eine effiziente Transformation von Bakterien zu ermöglichen, wurden die Bakterienstämme TOP10F' und JS5 mit einer nach Hanahan (1983) modifizierten Methode kompetent gemacht. Nach dem Animpfen von 100 ml LB-Medium mit 500 µl einer Übernachtkultur wurde die Bakteriensuspension bei 37° C bis zu einer Dichte (OD₅₅₀) von 0,6 inkubiert. Anschließend wurden die Bakterien auf Eis gekühlt, bei 6.000 rpm und 4° C pelletiert (Minifuge RF, Heraeus, Hanau) und in 40 ml TFB1-Puffer (30 mM KOAc, 100mM RbCl₂, 10 mM CaCl₂, 15 % Glycerin, pH 5,8 mit Essigsäure eingestellt, danach sterilfiltriert) resuspendiert. Nach einer Inkubationszeit von 15 Minuten auf Eis und einer Zentrifugation bei 6.000 rpm und 4° C wurde das Bakterienpellet in 4 ml TFB2-Puffer (10 mM MOPS, 75 mM CaCl₂, 10 mM RbCl₂, 15 % Glycerin, pH 6,5 mit KOH-Lösung eingestellt, danach sterilfiltriert)resuspendiert. Die Bakteriensuspension wurde dann in Aliquots je 100 µl aufgeteilt und auf Trockeneis schockgefroren. Die Lagerung erfolgte bei -70° C. Zur Transformation wurden 100 µl der kompetenten Bakterien auf Eis aufgetaut und nach Zugabe von 1-2 µl des jeweiligen Ligationsansatzes für 30 Minuten auf Eis inkubiert. Nach einem anschließenden Temperaturschock (45 s bei 42° C anschließend 2 min auf Eis) wurden die Bakterien mit 500 µl SOC-Medium (GIBCO/BRL, Eggenstein) vesetzt und bei 37° C für eine Stunde zur Expression der Antibiotikaresistenz in einem Bakterienschüttler kultiviert. Die Bakteriensuspension wurde auf LB-Agarplatten, die mit dem Antibiotikum Ampicillin supplementiert waren, ausgestrichen und bei 37° C über Nacht inkubiert.

Die Präparation von Plasmiden aus Bakterien (E.coli TopF10) erfolgte mit den QIAGEN-Plasmid-Kits der Firma QIAGEN, Hilden. Für die Präparation einer geringen Menge Plasmid-DNA wurden die Bakterien einer 15 ml-Übernachtkultur (LB-Medium mit 50 µg/ml Ampicillin) mit den vom Hersteller gelieferten Lösungen lysiert und über eine Anionenaustauscher-Säule (tip-20) gereinigt. Zur Gewinnung großer Mengen Plasmid-DNA (Maxi-Präparation) wurden 400 ml Übernachtkulturen angesetzt

### 3. Selektion der retroviraien Vektoren

Transiente Transfektion der scFv-pTC53-Expressionskonstrukte in die Verpackungszelle DSH-CXL mittels Elektroporation: Für die Elektroporation wurden jeweils 2 x 10⁶ DSH-CXL Zellen in 480 µl PBS resuspendiert und in eine auf Eis gelagerten Gene-Pulser-Küvette (0,4 cm Elektrode, Lücke 50, Biorad, München) überführt. Danach wurden 20 µg der rekombinanten Plasmid-DNA der Zellösung zugegeben. Der Inhalt der Küvette wurde einem elektrischen Puls in einem Elektroporator (Gene-Pulser Apparatus, Biorad, München) bei 270 V und 960 µF ausgesetzt. Nach einer 10-minutigen Inkubation der Küvette auf Eis wurden die Zellen in 20 ml frisches Kulturmedium in eine mittlere Zellkulturflasche (Nunc, Wiesbaden) überführt. Am nächsten Tag wurden die DSH-CXL-Zellen mit frischem Medium versetzt und kultiviert.

Der Virus-haltige Überstand der Transfektanden wurde zur Transduktion der Zielzellen eingesetzt. Am Tag vor der Transduktion wurden die C8166-Zielzellen im Verhältnis 1:2 in frisches Medium umgesetzt. Die Überstände wurden mit einem 0,45 µm Filter (Sartorius) steril filtriert. 7 ml des Überstandes wurden dann direkt zur Transduktion von 2x10⁵ C8166-Zellen eingesetzt. Um die Anbindung der retroviralen Vektoren an die Zelloberfläche zu stabilisieren erfolgte eine Zugabe von 40 µg/ml Polybren. Nach einer 2 stündigen Inkubation bei 37° C, wurden die Zellen mit PBS gewaschen und in frisches Kultur-Medium überführt.

Nachweis der β-Galaktosidase-Aktivität (X-Gal-Assay): Zur Überprüfung einer erfolgreichen Transduktion wurde nach 72 Stunden ein X-Gal-Assay nach der Methode von Sanes *et al.* (1986) modifiziert durchgeführt. Der Zellkulturüberstand wurde abgezogen und die Zellen mit PBS ohne (Ca²⁺ und Mg²⁺) gewaschen. Anschließend wurden die Zellen mit einer Fixierlösung (2 % Formaldehyd, 0,2 % Glutaraldehyd in PBS) fiir 5 min überschichtet und mit PBS gewaschen. Danach wurden die Zellen in 3 ml X-Gal-Reaktionsmixlösung (1 mg/ml, 5 mM K-Ferricyanid, 5 mM K-Ferrocyanid, 2mM MgCl₂) resuspendiert. Nach einer ca. 4-stündigen Inkubation des Ansatzes bei 37° C trat die Blaufärbung der transduzierten Zellen auf.

Von den 150 getesteten scFv-pTC53-Expressionskonstrukten waren 6 zellspezifisch (M8, K6, 7A5, 7E10, 6C3, 7B4). Das heißt es konnten pro zellspezifischem Konstrukt 10-20 blaugefärbte C8166-Zellen erkannt werden. Das ist im Vergleich zu nicht zellspezifischen scFv-Klonen signifikant. Von 6 zellspezifischen scFv-Expressionskonstrukten wurden stabile Verpackungszellinien generiert.

### 4. Etablierung stabiler Verpackungszellinien

Herstellung des Zeocin-Resistenzgens mittels PCR ausgehend von DNA des Plasmids pSCVZeo (Fa. Invitrogen): Um Verpackungszellen nach einer stabilen Transfektion mit dem pTC53zeo-scFv-Plasmid auf eine stabile Expression des Resistenzgens zu selektionieren, wurde eine Zeocin-Kassette integriert. Hierzu wurde mittels rekombinanter PCR aus dem Vektor pZeoSV2 (+/-) der Firma Invitrogen (NV Leek, Niederlande) eine Zeocin-Kassette amplifiziert und mit den Restriktionsschnittstellen Ndel 5'und 3' versehen, so daß die Kassette anschließend in den NdeI restringierten Anteil des pUC19-Rückgrades des pTC53 inseriert werden konnte. Der PCR-Ansatz (100 µl) enthielt: 1 x PCR-Puffer (Taq: 10 mM Tris/HCl, pH 8,8, 50 mM KCl, 1,5 mM MgCl₂, 0,01% Gelatine, 10 µM (+)- und 10 µM (-)-Primer, 200 µM je Desoxynukleotid, 2,5 Einheiten Taq-Polymerase und 100 ng Plasmid-DNA oder. Es wurden folgende Olgonukleotide verwendet.

Die PCR-Bedingungen waren: 94°C/3 min, [94°C/1 min, 60°C/1 min, 72°C/1,5 min.] 30 X Zyklen, 72°C/10 min und 4°C Endtemperatur.

Insertion eines Zeocin-Resistenz-Gen in die im transienten Test positiven scFv-pTC53-Env-Expressionskonstrukte.

Transfektion mittels Lipofectamin ™ (GIBCO/BRL, Life Technologies, Eggenstein).

Lipofectamin™ :N-[2-({2,5bis[-(3-aminopropyl)amino]-1-oxypemtyl}amino)ethyl]-N,N-dimethyl-2,3. bis (9-octadecenyloxy)-1-propanaminium trifluoroacetat) /Dioleoylphophatidylethanolamin; 3 : 1 (w/w)

Am Vortag der Transfektion wurden 1x10⁶ Zellen in eine 60 mm-Zellkulturschale (Greiner, Nürtingen) ausgesät. Für die Transfektion wurden 1-5 µg (je nach Versuchansatz) der rekombinanten Plasmid-DNA in 200 µl serumfreiem Medium resuspendiert. Parallel dazu wurden 8-25 µl (je nach Versuchansatz) Lipofectamin™ in 200 µl serumfeiem Medium verdünt. Nach dem Vereinigen beider Lösungen, folgte eine Inkubation für 45 min bei RT. Das DNA-Liposomen-Gemisch wurde auf 2 ml Endvolumen aufgefüllt und auf die mit serumfreiem Medium gewaschenen Zellen gegeben. Daraufhin wurden die Zellen für 5 Stunden bei 37° C inkubiert. Anschließend wurden 2 ml frisches Medium zugegeben, das die zweifache Konzentration an FCS enthielt. Am nächsten Tag erfolgte ein Mediumwechsel.

Zur Etablierung stabiler Verpackungszellklone wurden die Zellen 24 Stunden nach der Transfektion mit einem Selektionsmedium überschichtet. Das Zeocin™-Resistenzgen (*Streptoalloteichus hinductanus* Bleomycin-Gen) wurde als Selektionmarker benutzt. Die Selektion erfolgte in DMEM-Medium, supplementiert mit 525 µg/ml Zeocin™ (Phleomycin von *Streptomyces verticillus*; Invitrogen BV, Niederlande). Die Zellen wurden zweimal wöchentlich mit frischem Selektionsmedium versetzt. Nach ca. 4 Wochen konnten Zellfoci, die Zellklone darstellten, identifiziert werden. Diese Kolonien wurden einzel abgenommen, in eine 24-Loch-Platte (Flachboden, Nunc, Wiesbaden) in Zellkulturmedium ohne Antibiotikumssupplement überführt. Das Medium wurde zweimal wöchentlich gewechselt. Erlangten die Zellen eine Konfluenz von ca. 90 %, wurden sie in entsprechend größere Zellkulturgefäße expandiert.

## Patentansprüche

1. Verfahren zur Herstellung zellspezifischer retroviraler Vektoren, umfassend die folgenden Schritte:
a) Immunisieren eines Säugetiers mit einer oder mehreren Zellpopulation(en),
b) Isolieren von RNA aus dem immunisierten Säugetier, umfassend die B-Zell-RNA,
c) Herstellen von cDNA-Abschnitte der variablen Regionen der schweren und leichten Kette der Immunglobuline aus der isolierten RNA mittels RT-PCR mit Primern fiir die schwere und leichte Kette der Immunglobuline, wobei die Primer die Nukleinsäuresequenz für einen Oligopeptidlinker umfassen,
d) Ligieren der cDNA-Abschnitte zu scFv-cDNAs,
e) Ligieren der scFv-cDNAs in einen Phagemid-Vektor und Transformieren eines Wirtsbakteriums mit dem Phagemid-Vektor,
f) Isolieren von Phagen, die an die in Schritt a) verwendete(n) Zellpopulation(en) binden, durch Selektion,
g) Isolieren von zellspezifischen Phagen aus den in Schritt f) erhaltenen Phagen, die nur an die in Schritt a) verwendete(n) Zellpopulation(en) binden, durch Selektion,
h) Ausschneiden der scFv-codierenden DNA-Fragmente aus den in Schritt g) erhaltenen zellspezifischen Phagen und Ligieren in einen psi-negativen retroviralen Env-Expressionsvektor,
i) Transformieren des erhaltenen Env-scFv-Expressionsvektors in eine Verpackungszelle, und
j) Isolieren der von der Verpackungszelle sezernierten retroviralen Vektoren.

2. Verfahren nach Anspruch 1, wobei die in Schritt g) erhaltenen zellspezifischen Phagen vereinzelt werden

3. Verfahren nach Anspruch 1 oder 2, wobei die Schritte f) und/oder g) mindestens einmal wiederholt werden.

4. Verfahren nach einem der Ansprüche 1 bis 3, weiter umfassend den Schritt:
k) Isolieren der von der Verpackungszelle sezernierten retroviralen Vektoren, die die Zellen der Zellpopulation(en) transduzieren durch Selektion.

5. Verfahren nach einem der Ansprüche 1 bis 4, wobei das Säugetier ausgewählt ist aus der Gruppe Maus, Ratte, Meerschweinchen, Kaninchen, Ziege oder Schaf.

6. Verfahren nach einem der Ansprüche 1 bis 4, wobei die Zellpopulation(en) von Mensch, Maus, Ratte, Schaf, Rind oder Schwein stammen kann.

7. Verfahren nach Anspruch 6, wobei die Zellpopulation(en) ausgewählt ist (sind) aus der Gruppe, umfassend T-Zellen, Epithelzellen, Muskelzellen, hämatopoietische Zellen, Stammzellen, neurale Zellen, Karzinomzellen oder Leberzellen.

8. Verfahren nach einem der Ansprüche 1 bis 7, wobei das env-Gen des psi-negativen retroviralen Env-Expressionsvektors vom Milznekrosevirus (SNV) stammt.

9. Verfahren nach Anspruch 8, wobei der Expressionsvektor der Vektor mit der Bezeichnung pTC53 und der in Abb.4 dargestellten Nukleotidsequenz ist.

10. Retrovirale Vektoren enthaltend env- und scFv-Sequenzen, **dadurch gekennzeichnet dass** die scFv-Sequenzen gewonnen werden durch das Verfahren nach einem der Ansprüche 1-9, wobei die Zellpopulation(en) ausgewählt ist (sind) aus der Gruppe, umfassend T-Zellen, Epithelzellen, Muskelzellen, hämatopoietische Zellen, Stammzellen, neurale Zellen oder Leberzellen.

11. Retroviraler Vektor nach Anspruch 10 zur Verwendung als Arzneimittel.

12. Verwendung des Vektors nach Anspruch 10 zur Herstellung eines Arzneimittels zur somatischen Gentherapie, Impftherapie oder Diagnostik.

13. Verwendung des Vektors nach Anspruch 10 zur Herstellung eines Arzneimittels zur Therapie der Cystischen Fibrose, des ADA-Mangels, der chronischen Granulomatose oder der HIV-1 Infektion.

## Claims

1. A method for the production of cell-specific retroviral vectors comprising the following steps:
a) immunizing a mammal with one ore more cell population(s),
b) isolating RNA from the immunized mammal, comprising the B cell RNA,
c) production of cDNA regions of the variable regions of the immunoglobulin heavy and light chain from the isolated RNA by means of RT-PCR with primers for the immunoglobulin heavy and light chain wherein the primers comprise the nucleic acid sequence for an oligopeptide linker;
d) ligation of the cDNA regions to scFv-cDNAs,
e) ligation of the scFv-cDNAs into a phagemid-vector and transformation of a host bacterium with the phagemid vector,
f) isolation of phages binding to the cell populations(s) used in step a) by means of selection,
g) isolation of cell-specific phages from the phages obtained in step f) which only bind to the cell population(s) used in step a) by means of selection,
h) excision of the scFv-encoding DNA fragments from the cell-specific phages obtained in step g) and ligation into a psi-negative retroviral Env expression vector,
i) transformation of the resulting Env-scFv expression vector into a packaging cell, and
j) isolation of the retroviral vectors secreted by the packaging cell.

2. The method according to claim 1 wherein the cell-specific phages obtained in step g) are isolated.

3. The method according to claim 1 or 2 wherein the steps f) and/or g) are repeated at least once.

4. The method according to any of the claims 1 to 3 further comprising the step of:
k) isolating the retroviral vectors secreted by the packaging cell, which transduce the cells of the cell population(s) by means of selection.

5. The method according to any of the claims 1 to 4 wherein the mammal is selected from the group consisting of mouse, rat, guinea pig, rabbit, goat or sheep.

6. The method according to any of the claims 1 to 4 wherein the cell population(s) may originate from man, mouse, rat, sheep, cattle or pig.

7. The method according to claim 6 wherein the cell population(s) is/are selected from the group comprising T cells, epithelial cells, muscle cells, hematopoietic cells, stem cells, neural cells, carcinoma cells or liver cells.

8. The method according to any of the claims 1 to 7 wherein the env gene of the psi-negative retroviral Env expression vector is derived from spleen necrosis virus (SNV).

9. The method according to claim 8 wherein the expression vector is the vector having the designation pTC53 and the nucleotide sequence shown in Fig. 4.

10. Retroviral vectors containing env- and scFv-sequences, **characterized in that** said scFv-sequences are obtained by the method according to any one of claims 1 to 9, wherein the cell population(s) is/are selected from the group comprising T-cells, epithelial cells, muscle cells, hematopoietic cells, stem cells, neural cells and liver cells.

11. The use of the retroviral vector according to claim 10 as medicament.

12. The use of the vector according to claim 10 for the preparation of a medicament for somatic gene therapy, vaccination therapy or diagnostics.

13. The use of the vector according to claim 10 for the preparation of a medicament for the therapy of cystic fibrosis, ADA deficiency, chronic granulomatosis or HIV-1 infection.

## Revendications

1. Procédé pour la préparation de vecteurs rétroviraux à spécificité cellulaire, comportant les étapes suivantes:
a) immunisation d'un mammifère avec une ou plusieurs population(s) de cellules,
b) isolement d'ARN d'un mammifère immunisé, y compris l'ARN des cellules B,
c) préparation de segments d'ADNc des régions variables des chaînes lourdes et légères des immunoglobulines à partir de l'ARN isolé, au moyen de RT-ACP avec des amorces pour les chaînes lourdes et légères des immunoglobulines, tandis que les amorces comprennent la séquence d'acide nucléique pour un lieur oligopeptidique,
d) ligature des segments d'ADNc pour former des scFv-ADNc,
e) ligature dess scFv-ADNc dans un vecteur plasmidique et transformation d'une bactérie hôte avec le vecteur plasmidique,
f) isolement de phages, qui se lient à la(aux) population(s) cellulaire(s) utilisée(s) dans l'étape a), par sélection,
g) isolement de phages à spécificité cellulaire à partir des phages obtenus dans l'étape f), qui se lient seulement à la(aux) populations(s) cellulaire(s) utilisée(s) dans l'étape a), par sélection,
h) clivage des fragments d'ADN codant pour scFv à partir des phages à spécificité cellulaire obtenus dans l'étape g) et ligature dans un vecteur d'expression d'env rétroviral psi-négatif,
i) transformation du vecteur d'expression d'env-scFv obtenu, dans une cellule d'encapsidation, et
j) isolement des vecteurs rétroviraux sécrétés par la cellule d'encapsidation.

2. Procédé selon la revendication 1, dans lequel les phages à spécificité cellulaire obtenus dans l'étape g) sont isolés.

3. Procédé selon la revendication 1 ou 2, dans lequel les étapes f) et/ou g) sont répétées au moins une fois.

4. Procédé selon l'une des revendications 1 à 3, comportant en outre l'étape de:
k) isolement des vecteurs rétroviraux sécrétés par la cellule d'encapsidation, qui transduisent les cellules de la(les) population(s) de cellules par sélection.

5. Procédé selon l'une des revendications 1 à 4, dans lequel le mammifère est choisi dans le groupe des souris, rats, cochons d'Inde, lapins, chèvres ou moutons.

6. Procédé selon l'une des revendications 1 à 4, dans lequel la(les) population(s) de cellules peu(ven)t provenir d'humains, de souris, de rats, de moutons, de bovins ou de porcs.

7. Procédé selon la revendication 6, dans lequel la(les) population(s) de cellules est(sont) choisie(s) dans le groupe comprenant les cellules T, les cellules épithéliales, les cellules musculaires, les cellules hématopoïétiques, les cellules souches, les cellules nerveuses, les cellules de carcinome ou les cellules hépatiques.

8. Procédé selon l'une des revendications 1 à 7, dans lequel le gène env du vecteur d'expression d'env rétroviral psi-négatif provient du virus de nécrose de la rate (SNV).

9. Procédé selon la revendication 8, dans lequel le vecteur d'expression est le vecteur ayant la désignation pTC53 et la séquence nucléotidique représentée dans la Fig. 4.

10. Vecteurs rétroviraux contenant des séquences env et scFv, **caractérisés en ce que** les séquences scFv sont obtenues par le procédé selon l'une des revendications 1-9, tandis que la(les) population(s) est(sont) choisie(s) dans le groupe comprenant les cellules T, les cellules épithéliales, les cellules musculaires, les cellules hématopoïétiques, les cellules souches, les cellules nerveuses ou les cellules hépatiques.

11. Vecteur rétroviral selon la revendication 10 pour utilisation comme médicament.

12. Utilisation du vecteur selon la revendication 10 pour la préparation d'un médicament pour la thérapie génique somatique, la vaccinothérapie ou le diagnostic.

13. Utilisation du vecteur selon la revendication 10 pour la préparation d'un médicament pour la thérapie de la fibrose kystique, du déficit d'ADA, de la granulomatose chronique ou de l'infection à VIH-1.
